# EUROPEAN PATENT SPECIFICATION

(11) **EP 2 473 127 B1**
(45) Date of publication and mention of the grant of the patent: **20.05.2015**
(21) Application number: 10771330.7
(22) Date of filing: 12.10.2010
(51) Int. Cl.: A61B 17/78, A61B 17/72, A61B 17/74

(54) **AN ORTHOPEDIC NAIL AND AN ORTHOPEDIC NAIL SYSTEM**
ORTHOPÄDISCHER NAGEL UND ORTHOPÄDISCHES NAGELSYSTEM
CLOU ORTHOPÉDIQUE ET SYSTÈME DE CLOU ORTHOPÉDIQUE

(30) Priority: 13.10.2009 WO PCT/EP2009/007354
(43) Date of publication of application: 11.07.2012
(73) Proprietor: Zimmer GmbH, 8404 Winterthur (CH)
(72) Inventor: SCHWAMMBERGER, Andreas, 4435 Niederdorf (CH); VELIKOV, Jordan, 8810 Horgen (CH); DITTMANN, Rolf, 5415 Nussbaumen (CH)
(74) Representative: Manitz, Finsterwald & Partner GbR
(86) International application number: PCT/EP2010/006216
(87) International publication number: WO 2011/045025

(56) References cited:
- EP-A2- 1 016 382
- WO-A1-2008/147975
- DE-U1-202004 018 748
- GB-A- 2 209 947
- US-A1- 2004 172 027

## Description

The present disclosure relates to an orthopedic nail and an orthopedic nail system, in particular for the treatment of femoral neck fractures.

Intramedullary nails are known for the treatment of fractures of long bones. For example, known femoral nail systems include an intramedullary rod having a geometry adapted to that of the shape of the femoral bone and a lag screw for entering and fixating the femoral head to the main body of the femur should the femoral neck be fractured. The rod comprises a through bore suited for accommodating the lag screw, wherein the bore extends from a lateral and more distal position to a medial and more proximal position in a proximal section of the intramedullary rod. The angle between the rod axis and the through bore axis, defining, in an implanted state, also the lag screw axis, is chosen such that it is as good as possible in agreement with a patient's CCD angle. During surgery, the lag screw is typically fed through the through hole from a lateral side to a medial side of the intramedullary rod to accommodate the lag screw inside the through hole.

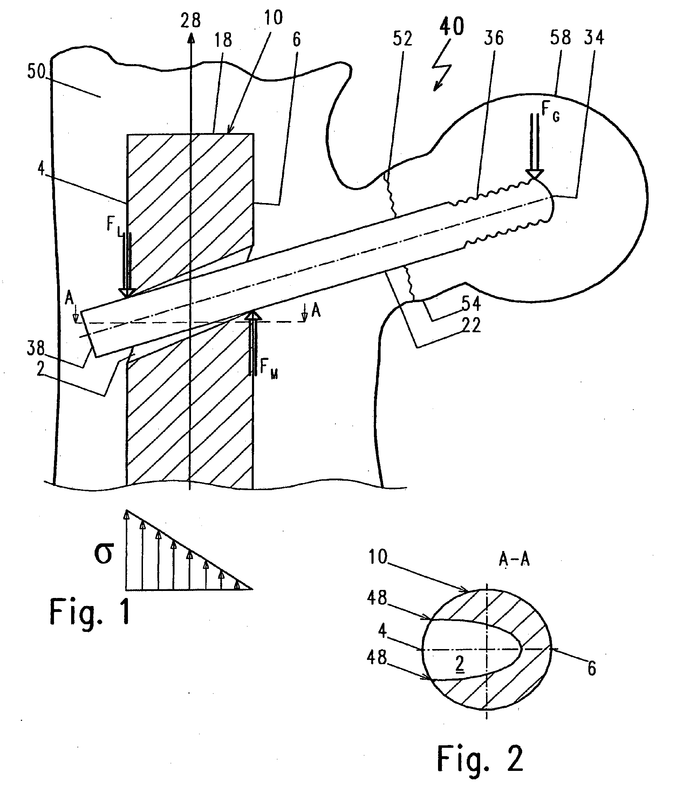

As shown in Fig. 1, in such a system, the proximal end of the lag screw is loaded with a force F_{G}. Generally, there is a certain amount clearance between the through hole and the lag screw to enable the lag screw to float axially inside the bore, in particular towards the lateral side, during the healing process. This clearance which leads to the lag screw essentially having two support points in the through hole, which bear the force F_{G} induced: one support point at the medial side of the intramedullary nail in the distal region of the through hole opening, where the force F_{M} acts on the lag screw, and a second support point at the lateral side of the intramedullary nail in the proximal region of the through hole opening, where the force F_{L} acts. This force F_{L} is amplified by the ratio of the leverage of the lag screw in the through hole. The force F_{M} generally induces a compressive stress in the massive distal part of the rod. The force F_{L} induces a tensile stress in the region of the through hole. The maximum tension is found in a region of the through hole having a very critical geometry and sharp edges. This, in combination with a notch effect of the through hole in the rod leads to an even worse tension distribution and a maximum tension is found on the lateral side of the through hole, where there is very little material provided to be able to bear this tension.

For this reason, it is known to build intramedullary rods with a very strong material and with a large amount of material in the region of the through hole. This increase of the rod size in the region of the through hole is both demanding in cost and work and also requires an extreme amount of healthy bone and tissue removal on insertion of the orthopedic nail system into the bone. WO 2008/ 147975 A1 discloses an intramedullary nail with an optimized reinforcement zone around the lateral end of the through hole.

EP 1 452 144 A2 discloses an intramedullary nail having a recess surrounding the lateral end of the through hole such that a flattened rim is provided. This recess has a large volume.

Documents WO 2008/ 147975 A1 and DE 20 2004 018 748 U1 disclose an intramedullary nail including an elgonated body having a transverse bore, wherein the transverse bore includes a substantially cylindrical portion, and slots and a groove, respectively.

One object of this disclosure is to provide a novel orthopedic nail that is able to cope with the above described adverse effects.

Accordingly, the present disclosure provides an orthopedic nail of generally rod-like shape. The orthopedic nail has a through hole. The through hole extends from a first side to a second side of the orthopedic nail. The through hole is adapted to receive a screw and/or a pin. A first end of the through hole at the first side defines a rim region. At least one notch is provided in the rim region. The notch is arranged transverse to a nail axis of the orthopedic nail. A width of the notch in the direction of the nail axis is smaller than a diameter of the through hole.

Accordingly, the present disclosure provides an orthopedic nail of generally and/or at least substantially rod-like shape. The orthopedic nail has a through hole. The through hole extends from a first side to a second side of the orthopedic nail. The through hole is adapted to receive a screw and/or a pin. A first end of the through hole at the first side defines a rim region. At least one surface notch, in particular at least a part thereof, in particular having a notch ground, is provided in the rim region at and restricted to an outer circumferential surface of the orthopaedic nail. In particular, the surface notch is arranged transverse to a nail axis of the orthopedic nail and/or of a proximal portion of the orthopedic nail. A width of the surface notch in the direction of the nail axis is smaller than a diameter of the through hole. In particular, the through hole extends transverse and/or obliquely to the nail axis.

The notch is suited to interrupt a high concentration of tensile stress lines in the rim region at the first side of the orthopedic nail, thereby shifting the high concentration of tensile stress and moving the maximum stress concentration to a location where more material is available. Thereby the rim region at the first side of the orthopedic nail does not have to be reinforced reducing the cost of the orthopedic nail. One advantage here as compared to e.g. EP 1 452 144 A2 is that the bone ingrowth volume is reduced and hence an additional trauma on extraction of the orthopedic nail is considerably reduced.

In an embodiment, the surface notch is provided at and restricted to the outer circumferential surface of the orthopaedic nail, i.e. does not fully extend through the orthopaedic nail.

In an embodiment, when viewed from the first side and/or in a plan view onto the first side, a depth of the surface notch along the viewing direction and terminating in the notch ground is smaller than the diameter of the orthopaedic nail. Thus, the surface notch has a finite depth. The depth of the notch may be smaller than or equal to a half, in particular a third or a fourth or a fifth, of the diameter of the orthopaedic nail. The depth of the notch may be equal to or smaller than 4 mm, in particular 3 mm or 2 mm.

In an embodiment, when viewed from an anterior or posterior side, the depth of the surface notch along the first-side-viewing direction is defined by the distance between the outermost point of the outer circumferential surface of the in particular virtual none-holed orthopaedic nail and the innermost point of the notch ground.

The slots and the groove of documents 2008/147975 A1 and DE 20 2004 018 748 U1, respectively, are not provided to enhance the strength of the intramedullary nail in the region of the transverse bore. When viewed from a side at which an end of the transverse bore is provided, the slots and the groove do not have a ground and, thus, no associated depth along that viewing direction, but instead fully extend through the intramedullary nail. Thus, the surface notch according to the present disclosure is substantially different from the slots and the groove disclosed in documents 2008/147975 A1 and DE 20 2004 018 748 U1, respectively.

In an embodiment, the notch is intersected by and/or superimposed with the first end of the through hole. Depending on its proximal-distal position, the notch may be visible partly only and/or a partly virtual notch. In particular, only an anterior portion and a posterior portion of the notch may be visible, in particular wherein the anterior and posterior portions are not connected to each other but separated by the first end of the through hole, the anterior and posterior portions of the notch being provided at an anterior part of the rim region anterior of a proximal-distal center plane of the through hole and a posterior part of the rim region posterior of the proximal-distal center plane of the through hole.

In a further embodiment, the surface notch and/or the notch ground is of longitudinal shape, a length of the surface notch and/or the notch ground extending, in particular in a transverse plane, tangentially to, in a direction parallel offset to a tangent to or at least partly circumferentially around the outer circumferential surface. The surface notch may be an elongate surface notch. The elongate surface notch may be visible partly only as mentioned above. The surface notch and/or a length of the surface notch may be arranged transverse to a through hole axis of the through hole and/or at a distance from the through hole axis.

In a further embodiment, in a plan view onto the first side, the surface notch exceeds the through hole and/or the rim region in anterior direction and posterior direction.

In a further embodiment, the notch is arranged in parallel to a plane which is substantially perpendicular to the nail axis.

In a further embodiment, the notch is of longitudinal shape and/or a length of the notch is longer than the diameter of the through hole.

In a further embodiment, the notch is arranged substantially perpendicular to a through hole axis of the through hole and/or is arranged substantially perpendicular to the nail axis. A length of the notch may be oriented in anterior-posterior direction of the orthopedic nail. As to a definition of the anterior-posterior direction, it becomes clear to the skilled person, as the nail has a proximal distal direction essentially along its elongation, a lateral-medial direction, essentially defined by a projection of the through bore axes on the nail cross section. The anterior-posterior direction is then defined as being arranged perpendicular to a plane which is spanned by the proximal-distal and the lateral-medial direction.

In a further embodiment, the width of the notch, e.g. the smaller dimension in a plan view onto the notch, is smaller than a radius of the through hole. A depth and/or the width of the notch may be in the range of 1 mm to 4 mm, wherein the lower limit of the range may as well be 1.5 mm and/or the upper limit may as well be 2 mm or 3 mm. The depth and/or the width of the notch may be up to a quarter, in particular up to a sixth or an eighth of the diameter of the through hole.

In an further embodiment, the notch is positioned in a proximal part of the rim region towards a proximal end of the orthopedic nail away from the through hole axis, in particular in a proximal third of the rim region.

In a further embodiment, the notch is provided at a proximal end of the rim region.

In a further embodiment, the notch is groove-shaped and/or a length of the notch has a cross-section which is of substantially rounded shape.

In a further embodiment, the notch is provided distant from a proximal end of the rim region. This might be beneficial, as the lateral support point is thereby not reduced in size and/or manipulated in shape and the above described leverage is not changed.

In a further embodiment, at least two notches are provided in the rim region.

In a further embodiment, a first notch is positioned in a proximal half of the rim region and a second notch is positioned in a distal half of the rim region.

In a further embodiment, a first notch is provided at a proximal end of the rim region and/or a second notch is provided at a distal end of the rim region. Thus, the region of low material strength and/or unfavorable geometry is isolated from the rest of the orthopedic nail which effectively prevents the region of low material strength from being charged with tensile or pressure stress in a direction parallel to the rod axis.

In a further embodiment, the notch is provided as at least one circumferential notch around the circumference of the orthopedic nail, wherein the circumferential notch is provided in a plane that is substantially perpendicular to the nail axis or the circumferential notch is provided in a plane oblique to the nail axis.

The orthopedic nail is an intramedullary nail adapted for the use within a bone, in particular a long bone, having a medullary channel.

In a further embodiment, the notch is provided on a lateral side of the orthopedic nail.

In a further embodiment, the surface notch is suited to interrupt tensile stress lines in the rim region induced by a force acting on a support point for the screw and/or pin at the first side in a proximal region of the first end of the through hole.

According to an aspect of the present disclosure, there is provided an orthopedic nail system in particular for the use as a femoral osteosynthetic system which includes an orthopedic nail in accordance with the present disclosure and a screw and/or a pin, wherein the screw and/or pin are adapted to be received in the through hole of the orthopedic nail and are also adapted for the treatment of bone fractures.

It will be appreciated that the-specific features of the embodiments described above can be combined. Thus any combinations of the features described in the dependent claims are disclosed herein, be they explicitly mentioned or not.

Further areas of applicability will become apparent from the description provided herein.

The present disclosure will be explained in more detail and become fully understood from the detailed description and the accompanying drawings, wherein
- Fig. 1: shows a longitudinal cross-section of an orthopedic nail system including an orthopedic nail according to the prior art,
- Fig. 2: shows a section along A-A in Fig. 1,
- Fig. 3: shows a side view of the orthopedic nail in Fig. 1,
- Figs. 4a and 4b: show an embodiment of an orthopedic nail,
- Figs. 5a and 5b: show a further embodiment of an orthopedic nail,
- Figs. 6a and 6b: show a further embodiment of an orthopedic nail, and
- Figs. 7a and 7b: show a further embodiment of an orthopedic nail.

The following description of the embodiments is merely exemplary in nature and is in no way intended to limit the disclosure, its application, or uses. It should be understood that throughout the drawings, the same reference numerals indicate similar or corresponding parts and features.

Fig. 1 already discussed above shows a fragmentary view of a prior art orthopedic nail system 40 in which the bone nail system or orthopedic nail system 40 is shown implanted into a femur 50. The orthopedic nail system 40 includes an orthopedic nail 10, which is an intramedullary rod or intramedullary nail 10. Also shown is a bone screw 22, which is also known as a lag screw 22. The lag screw 22 has a threaded portion 36 at its proximal end 34, and is typically inserted into the nail 10 from a first, lateral side 4 of the nail 10 through a through hole 2 of the orthopedic nail 10 and projects out of the nail 10 on a second, medial side 6 of the nail 10 into a femoral head 58 where it is subsequently anchored to aid in the treatment of a fracture 52 of a femoral neck 54 of the femur 50.

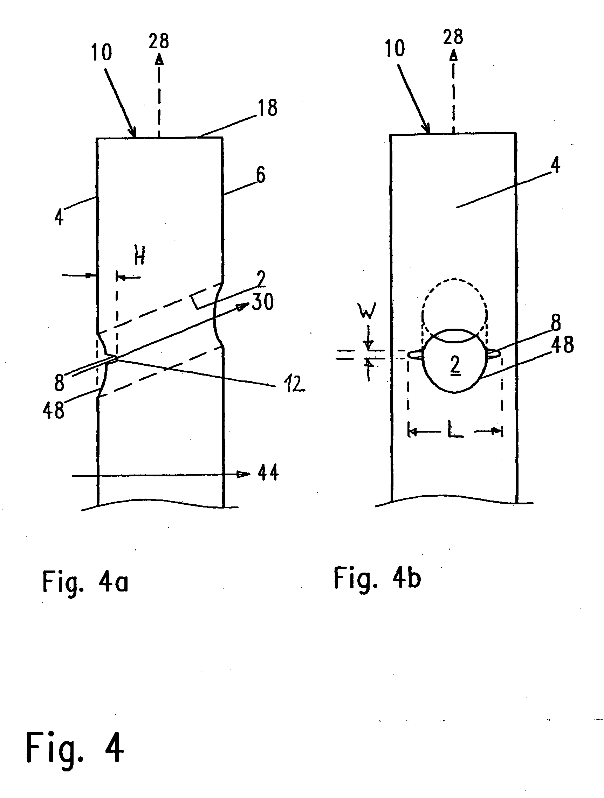

The through hole 2 is provided in a proximal part of the intramedullary nail 10, with the through hole 2 obliquely extending through the nail 10, wherein the angle between a through hole axis 30 as shown in Fig. 4 and a nail axis 28 is chosen such that it is in agreement with a patient's CCD angle. The lateral end of the trough hole 2 is located closer to a distal end of the nail 10 than the medial end of the through hole 2.

The through hole 2 may be a bore and may be produced by drilling the bore 2 into the nail 10. Fig. 1 only shows a proximal part of the nail 10 including a proximal end 18, as the distal part of the nail 10 may vary. Moreover, the type of lag screw 22 used may vary and is by no means limited to the screw 22 depicted in Fig. 1 but it may be any type of screw or pin used in this type of medical application. In particular, a distal end 38 of the lag screw 22 may be of any kind and shape as is typically used in this sort of application.

Arrow F_{G} indicates a the force acting on the distal end of the lag screw 22 due to loading by the patient's body weight, inducing a momentum on the lag screw through a lever arm at the distal part of the lag screw. Due to the clearance between the lag screw and the bore the lag screw is supported essentially at two support points, one at the medial distal end of the bore where force F_{M} acts on the lag screw, and at the lateral proximal end of the bore by Force F_{L}, whereby the latter has to provide an equilibrium for the momentum induced by F_{G}, with the medial support point as the pivotal point. While force F_{M} introduces a compressive stress component in the region of the nail distal from the bore, F_{L} induces merely tensile stresses in the region of the bore.

An theoretical qualitative distribution of the tensile stresses across the nail 10 due to the force exerted by lag screw 22, leaving any notch effect out of consideration, is shown in the lower part of Fig. 1. As is shown in combination with Fig. 2, which shows a section along line A-A of Fig. 1, the maximum tension is found in a rim region 48 which is defined by the lateral end of the through hole 2 which has a very critical geometry and sharp edges. The rim region 48 confines the lateral end of the through hole 2, i.e. confines the lateral through hole opening.

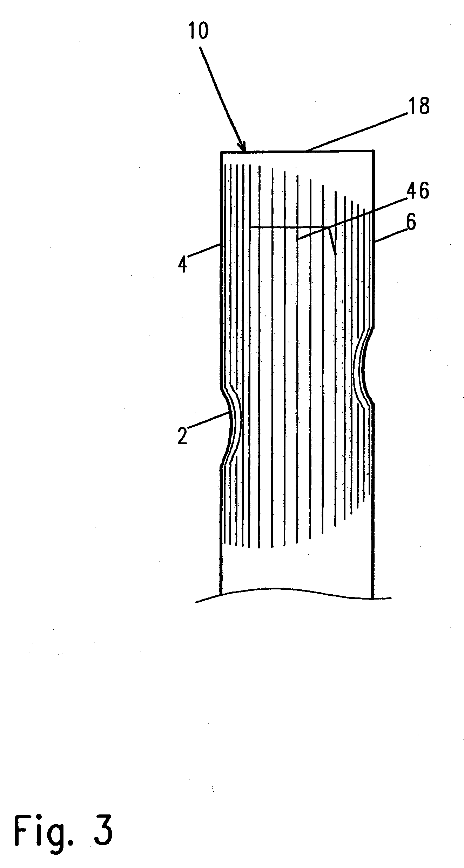

This tensile stress induces a "flux" of tension which "flux" of tension, due to the effect of the bore, concentrates in the rim region 48 of the through hole 2 and, thus, in a region of very low material strength. Fig. 3 shows schematic "tension flux" lines 46. On both ends of the through hole 2 the flux lines 46 concentrate in the region of the through hole 2, wherein on the lateral side 4 of the orthopedic nail 10 the flux lines 46 combine with the high tensile stress shown in Fig. 1. Thus, the tensile stresses are exaggerated where the lowest material strength is found, i.e. in the rim region 48.

In the following reference is made to Figures 4 through 7. It has been found that a transverse notch 8, 26 applied at the rim region 48 at the lateral end of the oblique through hole 2 is suited to interrupt the stress lines at the rim region 48, so as to virtually isolate the edge region of the bore from the tension flux and move the maximum stress concentration to a location where more material is available. Through the notch 8, 26, the region of low material strength and unfavorable geometry is virtually isolated from the tensile stress. In particular, the notch 8, 26 is provided at an outer wall of the orthopedic nail 10. The notch 8, 26 is a surface notch and, thus, is provided in the rim region 48 at and restricted to an outer circumferential surface of the orthopaedic nail 10. The surface notch 8, 26 terminates in a notch ground 12. One advantage here is, as compared to e.g. EP 1 452 144 A2, that the bone ingrowth volume and hence an additional trauma on the subsequent extraction of the nail is considerably reduced, and the extraction of the nail from the patient's body is facilitated.

The notch 8, 26 may be arranged virtually everywhere along the proximal-distal direction on the lateral end of the through hole 2. The notch 8, 26 may be e.g. drilled or milled. The notch 8, 26 will typically have a rounded cross-section, but as the case may be it can also have a triangular cross-section, a rectangular cross-section or any substantially concavely shaped cross-section. The notch 8, 26 can also be described as a groove.

Preferably both a width and a depth of the notch 8, 26 are in a range from 1 mm or 1.5 mm to 2 mm, 3 mm or 4 mm. In particular, the width of the notch 8, 26 corresponds to a dimension of the notch 8, 26 in the direction of the nail axis 28, and the depth of the notch 8, 26 corresponds to a dimension of the notch 8, 26 radially to the nail axis 28. As can be seen, the width of the notch 8, 26 is substantially smaller than a diameter of the through hole 2, e.g. the width of the notch 8, 26 may be approximately a sixth of the diameter of the through hole 2 or less. In choosing the geometry of the notch care should be taken in order not to introduce an excessive additional notch effect. The depth H and the width W of the notch 8, 26 are shown in Figs. 4a and 4b.

It may be found advantageous to provide a notch 8 as an elongate notch 8 transverse to the nail elongation and to arrange the notch 8 in the proximal half of the rim region 48 as shown in Figs. 4a and 4b, or in the proximal third of the rim region 48. As can be seen, the notch 8 is intersected by the lateral end of the through hole 2. Further, a plane 44 is indicated, which is substantially perpendicular to the nail axis 28. Thus, the through hole axis 30 is arranged obliquely to the plane 44. A length L of the notch, denominating the longer dimension of the notch may be at least as long as or longer than the diameter of the through hole 2 of the orthopedic nail 10. Fig. 4b shows the orthopedic nail 10 as is indicated in Fig. 4a, however, viewed from the lateral side 4. The notch 8 is perpendicular to the nail axis 28, is perpendicular to the through hole axis 30, and its longitudinal extension is arranged in parallel to the plane 44. Thus, the notch 8 and its notch ground 12 may be of longitudinal shape and extend with its length tangentially to and/or in a direction parallel offset to a tangent to the outer circumferential surface of the orthopaedic nail 10.

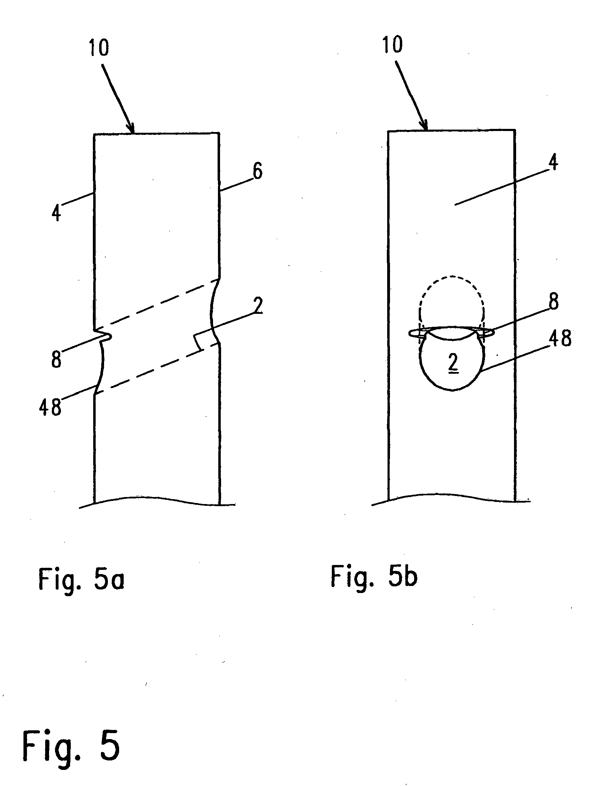

The embodiment in Figs. 5a and 5b shows a notch 8 arranged immediately at the proximal end of the rim region 48. It might prove advantageous while not mandatory in realizing the general idea of this invention to arrange the notch 8 distant from the proximal end of the rim region 48, and thus, to not change the leverage in supporting the momentum induced by the forces acting on the distal part of the lag screw 22.

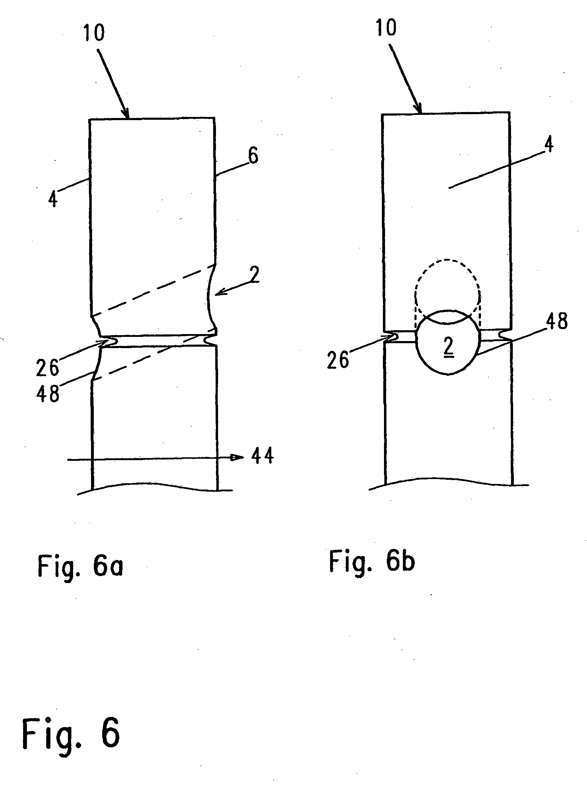

In one embodiment, the notch 26 is a circumferential notch 26 and runs around the nail 10 as is shown in Figs. 6a and 6b. On the lateral side 4 of the orthopedic nail 10, the notch 26 is provided in the proximal half of the rim region 48. On the medial side 6 of the orthopedic nail 10, the notch 26 is, in this specific embodiment, provided in a region distally to the medial end of the through hole 2. The circumferential notch 26, in this embodiment, is substantially parallel to the plane 44. Thus, the notch 26 and its notch ground 12 may be of longitudinal shape and extend with its length, in particular curved length, at least partly circumferentially around said outer circumferential surface of the orthopaedic nail 10.

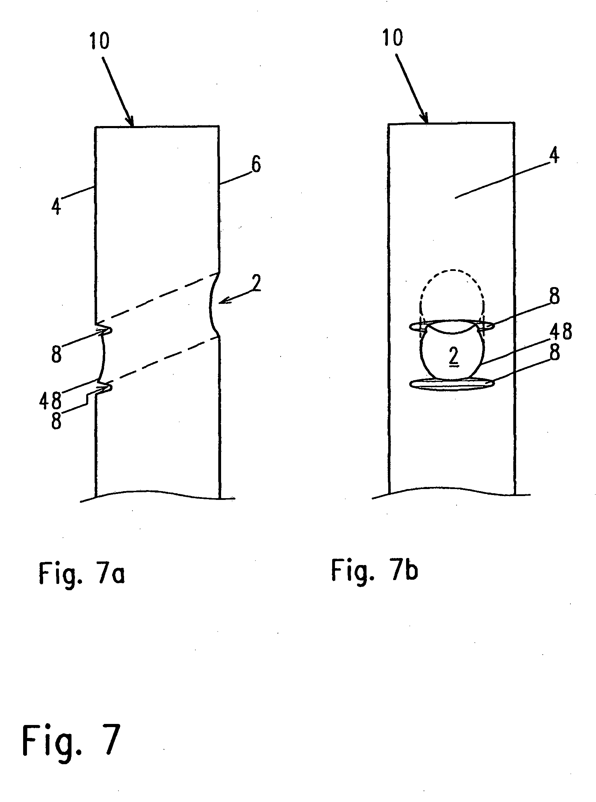

Figs. 7a and 7b show a further embodiment of the orthopedic nail 10. In this case, two notches 8 are provided on the lateral side 4 of the orthopedic nail 10. The more proximal one of the two notches 8 is situated at or close to the proximal end of the lateral end of the through hole 2, and the more distal one of the two notches 8 is provided at or close to the distal end of the lateral end of the through hole 2. This completely isolates the critical rim region 48 from the rest of the nail 10 with respect to stress components in the axial direction of the nail and thus effectively prevents the rim region 48 from being charged with tensile or pressure stress in a direction parallel to the nail axis.

While the present invention has been illustrated by the description of one or more embodiments thereof, and while the embodiments have been described in considerable detail, they are not intended to restrict or in any way limit the scope of the appended claims to such detail. Additional advantages and modifications will readily appear to those skilled in the art. The invention is defined in the claims.

### List of Reference Numerals:

- 2: through hole
- 4: lateral side
- 6: medial side
- 8: notch
- 10: intramedullary nail
- 12: notch ground
- 18: proximal end
- 22: bone screw
- 26: circumferential notch
- 28: nail axis
- 30: through hole axis
- 34: proximal end of bone screw
- 36: threaded portion of bone screw
- 38: distal end of bone screw
- 40: intramedullary nail system
- 44: plane
- 46: force lines
- 48: rim region
- 50: femur
- 52: fracture
- 54: femoral neck
- 58: femoral head

- H: Depth
- L: Length
- W: Width

## Claims

1. An intramedullary nail (10) of generally rod-like shape having a through hole (2) extending from a first side (4) to a second side (6) of said intramedullary nail (10) and being adapted to receive a screw (22) and/or a pin, a first end of said through hole (2) at said first side (4) defining a rim region (48), wherein at least one surface notch (8; 26) is provided in said rim region (48) at and restricted to an outer circumferential surface of said intramedullary nail (10), said surface notch (8; 26) being arranged transverse to a nail axis (28) of said intramedullary nail (10), **characterised in that** a width (W) of said surface notch (8; 26) in the direction of said nail axis (28) is smaller than a diameter of said through hole (2).

2. The intramedullary nail (10) in accordance with claim 1, wherein, when viewed from said first side (4), a depth (H) of said surface notch (8; 26) along the viewing direction and terminating in a notch ground (12) is smaller than the diameter of said intramedullary nail (10), in particular smaller than or equal to a half, in particular a third or a fourth or a fifth, of the diameter of the intramedullary nail (10).

3. The intramedullary nail (10) in accordance with claim 1 or claim 2, wherein said surface notch (8; 26) is intersected by said first end of said through hole (2).

4. The intramedullary nail (10) in accordance with any one of the preceding claims, wherein said surface notch (8; 26) and/or a notch ground (12) is of longitudinal shape, a length of said surface notch (8; 26) and/or said notch ground (12) extending tangentially to, in a direction parallel offset to a tangent to or at least partly circumferentially around said outer circumferential surface.

5. The intramedullary nail (10) in accordance with any one of the preceding claims, wherein said surface notch (8; 26) is arranged in parallel to a plane (44) which is at least substantially perpendicular to said nail axis (28).

6. The intramedullary nail (10) in accordance with any one of the preceding claims, wherein said surface notch (8) is of longitudinal shape and/or a length of said surface notch (8; 26) is longer than said diameter of said through hole (2), and/or wherein said width (W) of said surface notch (8; 26) is smaller than a radius of said through hole (2).

7. The intramedullary nail (10) in accordance with any one of the preceding claims, wherein said surface notch (8) is arranged at least substantially perpendicular to a through hole axis (30) of said through hole (2) and/or is arranged at least substantially perpendicular to said nail axis (28).

8. The intramedullary nail (10) in accordance with any one of the preceding claims, wherein said surface notch (8; 26) is positioned in a proximal part of said rim region (48) towards a proximal end (18) of said intramedullary nail (10) away from said through hole axis (30), in particular in a proximal third of said rim region (48).

9. The intramedullary nail (10) in accordance with any one of the preceding claims, wherein said surface notch (8; 26) is provided at a proximal end of said rim region (48) or wherein said surface notch (8; 26) is provided distant from a proximal end of said rim region (48).

10. The intramedullary nail (10) in accordance with any one of the preceding claims, wherein at least two surface notches (8; 26) are provided in said rim region (48).

11. The intramedullary nail (10) in accordance with claim 10, wherein a first surface notch (8; 26) is positioned in a proximal half of said rim region (48), and a second surface notch (8; 26) is positioned in a distal half of said rim region (48), and/or wherein a first surface notch (8; 26) is provided at a proximal end of said rim region (48) and/or a second surface notch (8; 26) is provided at a distal end of said rim region (48).

12. The intramedullary nail (10) in accordance with any one of the preceding claims, wherein said surface notch (26) is provided as at least one circumferential surface notch (26) around a circumference of said intramedullary nail (10), and wherein said circumferential surface notch (26) is provided in a plane (44) that is at least substantially perpendicular to said nail axis (28) or said circumferential surface notch (26) is provided in a plane obique to said nail axis (28).

13. The intramedullary nail (10) in accordance with any one of the preceding claims, wherein said surface notch (8; 26) is provided on a lateral side (4) of said intramedullary nail (10).

14. The intramedullary nail (10) in accordance with any one of the preceding claims, wherein said surface notch (8; 26) is suited to interrupt tensile stress lines in said rim region (48) induced by a force (F_{L}) acting on a support point for said screw (22) and/or pin at said first side (4) in a proximal region of said first end of said through hole (2).

15. An intramedullary nail system (40) in particular for use as a femoral osteosynthetic system including an intramedullary nail (10) in accordance with any one of the preceding claims and a screw (22) and/or a pin, wherein said screw and/or pin are adapted to be received in said through hole (2) of said intramedullary nail (10) and are adapted for the treatment of bone fractures.

## Patentansprüche

1. Marknagel (10) mit allgemein stangenartiger Form, der ein Durchgangsloch (2) aufweist, das sich von einer ersten Seite (4) zu einer zweiten Seite (6) des Marknagels (10) erstreckt und derart angepasst ist, eine Schraube (22) und/oder einen Stift aufzunehmen, wobei ein erstes Ende des Durchgangslochs (2) an der ersten Seite (4) einen Randbereich (48) definiert, wobei zumindest eine Oberflächenkerbe (8; 26) in dem Randbereich (48) an einer Außenumfangsfläche des Marknagels (10) vorgesehen und auf diese beschränkt ist, wobei die Oberflächenkerbe (8; 26) quer zu einer Nagelachse (28) des Marknagels (10) angeordnet ist,
**dadurch gekennzeichnet, dass**
eine Breite (W) der Oberflächenkerbe (8; 26) in der Richtung der Nagelachse (28) kleiner als ein Durchmesser des Durchgangsloches (2) ist.

2. Marknagel (10) nach Anspruch 1,
wobei bei Betrachtung von der ersten Seite (4) eine Tiefe (H) der Oberflächenkerbe (8; 26) entlang der Betrachtungsrichtung, und die in einem Kerbboden (12) ended, kleiner als der Durchmesser des Marknagels (10) ist, und insbesondere kleiner als oder gleich einer Hälfte, insbesondere einem Drittel oder einem Viertel oder einem Fünftel des Durchmessers des Marknagels (10) ist.

3. Marknagel (10) nach einem der Ansprüche 1 oder 2,
wobei die Oberflächenkerbe (8; 26) durch das erste Ende des Durchgangsloches (2) überschnitten ist.

4. Marknagel (10) nach einem der vorhergehenden Ansprüche,
wobei die Oberflächenkerbe (8; 26) und/oder ein Kerbboden (12) eine Längsform besitzen, wobei sich eine Länge der Oberflächenkerbe (8; 26) und/oder des Kerbbodens (12) tangential zu der äußeren Umfangsfläche, in einer Richtung parallel versetzt zu einer Tangente zu der äußeren Umfangsfläche oder zumindest teilweise umfangsmäßig um die äußere Außenumfangsfläche erstreckt.

5. Marknagel (10) nach einem der vorhergehenden Ansprüche,
wobei die Oberflächenkerbe (8; 26) parallel zu einer Ebene (44) angeordnet ist, die zumindest im Wesentlichen rechtwinklig zu der Nagelachse (28) liegt.

6. Marknagel (10) nach einem der vorhergehenden Ansprüche,
wobei die Oberflächenkerbe (8) eine Längsform besitzt und/oder eine Länge der Oberflächenkerbe (8; 26) länger als der Durchmesser des Durchgangsloches (2) ist, und/oder wobei die Breite (W) der Oberflächenkerbe (8; 26) kleiner als ein Radius des Durchgangsloches (2) ist.

7. Marknagel (10) nach einem der vorhergehenden Ansprüche,
wobei die Oberflächenkerbe (8) zumindest im Wesentlichen rechtwinklig zu einer Durchgangslochachse (30) des Durchgangsloches (2) angeordnet ist und/oder zumindest im Wesentlichen rechtwinklig zu der Nagelachse (28) angeordnet ist.

8. Marknagel (10) nach einem der vorhergehenden Ansprüche,
wobei die Oberflächenkerbe (8; 26) in einem proximalen Teil des Randbereiches (48) in Richtung eines proximale Endes (18) des Marknagels (10) weg von der Durchgangslochachse (30), insbesondere in einem proximalen Drittel des Randbereiches (48) positioniert ist.

9. Marknagel (10) nach einem der vorhergehenden Ansprüche,
wobei die Oberflächenkerbe (8; 26) an einem proximalen Ende des Randbereiches (48) vorgesehen ist oder wobei die Oberflächenkerbe (8; 26) entfernt von einem proximalen Ende des Randbereiches (48) vorgesehen ist.

10. Marknagel (10) nach einem der vorhergehenden Ansprüche,
wobei zumindest zwei Oberflächenkerben (8; 26) in dem Randbereich (48) vorgesehen sind.

11. Marknagel (10) nach Anspruch 10,
wobei eine erste Oberflächenkerbe (8; 26) in einer proximalen Hälfte des Randbereiches (48) positioniert ist und eine zweite Oberflächenkerbe (8; 26) in einer distalen Hälfte des Randbereiches (48) positioniert ist, und/oder wobei eine erste Oberflächenkerbe (8; 26) an einem proximalen Ende des Randbereiches (48) vorgesehen ist und/oder eine zweite Oberflächenkerbe (8; 26) an einem distalen Ende des Randbereiches (48) vorgesehen ist.

12. Marknagel (10) nach einem der vorhergehenden Ansprüche,
wobei die Oberflächenkerbe (26) als zumindest eine Umfangsoberflächenkerbe (26) um einen Umfang des Marknagels (10) vorgesehen ist, und wobei die Umfangsoberflächenkerbe (26) in einer Ebene (44) vorgesehen ist, die zumindest im Wesentlichen rechtwinklig zu der Nagelachse (28) liegt, oder die Umfangsoberflächenkerbe (26) in einer Ebene schräg zu der Nagelachse (28) vorgesehen ist.

13. Marknagel (10) nach einem der vorhergehenden Ansprüche,
wobei die Oberflächenkerbe (8; 26) an einer lateralen Seite (4) des Marknagels (10) vorgesehen ist.

14. Marknagel (10) nach einem der vorhergehenden Ansprüche,
wobei die Oberflächenkerbe (8; 26) geeignet ist, Zugspannungslinien in dem Randbereich (48) zu unterbrechen, die durch eine Kraft (F_{L}) bewirkt werden, die auf einen Abstützpunkt für die Schraube (22) und/oder den Stift an der ersten Seite (4) in einem proximalen Bereich des ersten Endes des Durchgangsloches (2) wirkt.

15. Marknagelsystem (40) insbesondere zur Verwendung als ein osteosynthetisches Femursystem, das einen Marknagel (10) nach einem der vorhergehenden Ansprüche und eine Schraube (22) und/oder einen Stift aufweist, wobei die Schraube und/oder der Stift zur Aufnahme in dem Durchgangsloch (2) des Marknagels (10) angepasst sind und zur Behandlung von Knochenbrüchen angepasst sind.

## Revendications

1. Clou intramédullaire (10) généralement en forme de tige ayant un trou traversant (2) s'étendant depuis un premier côté (4) jusqu'à un second côté (6) dudit clou intramédullaire (10) et étant adapté à recevoir une vis (22) et/ou une broche, une première extrémité dudit trou traversant (2) sur ledit premier côté (4) définissant une région de bordure (48), dans lequel au moins une encoche de surface (8 ; 26) est prévue dans ladite région de bordure (48) au niveau de et restreinte à une surface circonférentielle extérieure dudit clou intramédullaire (10), ladite encoche de surface (8 ; 26) étant agencée transversalement à un axe (28) dudit clou intramédullaire (10),
**caractérisé en ce qu'**une largeur (W) de ladite encoche de surface (8 ; 26) dans la direction dudit axe (28) du clou est plus petite qu'un diamètre dudit trou traversant (2).

2. Clou intramédullaire (10) selon la revendication 1 dans lequel, lorsqu'on l'observe depuis ledit premier côté (4), une profondeur (H) de ladite encoche de surface (8 ; 26) le long de la direction d'observation et se terminant dans un fond d'encoche (12) est plus petite que le diamètre dudit clou intramédullaire (10), en particulier plus petite ou égale à une moitié, en particulier un tiers ou un quart ou un cinquième, du diamètre du clou intramédullaire (10).

3. Clou intramédullaire (10) selon la revendication 1 ou 2, dans lequel ladite encoche de surface (8 ; 26) est recoupée par ladite première extrémité dudit trou traversant (2).

4. Clou intramédullaire (10) selon l'une quelconque des revendications précédentes, dans lequel ladite encoche de surface (8 ; 26) et/ou un fond d'encoche (12) présente une forme longitudinale, une longueur de ladite encoche de surface (8 ; 26) et/ou dudit fond d'encoche (12) s'étend tangentiellement à ladite surface circonférentielle extérieure, ou bien dans une direction parallèle et décalée à une tangente à ladite surface circonférentielle extérieure, ou encore au moins partiellement circonférentiellement autour de ladite surface circonférentielle extérieure.

5. Clou intramédullaire (10) selon l'une quelconque des revendications précédentes, dans lequel ladite encoche de surface (8 ; 26) est agencée parallèlement à un plan (44) qui est au moins sensiblement perpendiculaire audit axe (28) du clou.

6. Clou intramédullaire (10) selon l'une quelconque des revendications précédentes, dans lequel ladite encoche de surface (8) présente une forme longitudinale et/ou une longueur de ladite encoche de surface (8 ; 26) est plus longue que ledit diamètre dudit trou traversant (2), et/ou dans lequel ladite largeur (W) de ladite encoche de surface (8 ; 26) est plus petite qu'un rayon dudit trou traversant (2).

7. Clou intramédullaire (10) selon l'une quelconque des revendications précédentes, dans lequel ladite encoche de surface (8) est agencée au moins sensiblement perpendiculaire à un axe (30) dudit trou traversant (2) et/ou est agencée au moins sensiblement perpendiculaire audit axe (28) du trou.

8. Clou intramédullaire (10) selon l'une quelconque des revendications précédentes, dans lequel ladite encoche de surface (8 ; 26) est positionnée dans une partie proximale de ladite région de bordure (48) vers une extrémité proximale (18) dudit clou intramédullaire (10) en éloignement dudit axe (30) du trou, en particulier dans un tiers proximal de ladite région de bordure (48).

9. Clou intramédullaire (10) selon l'une quelconque des revendications précédentes, dans lequel ladite encoche de surface (8 ; 26) est prévue à une extrémité proximale de ladite région de bordure (48), ou dans lequel ladite encoche de surface (8 ; 26) est prévue à distance d'une extrémité proximale de ladite région de bordure (48).

10. Clou intramédullaire (10) selon l'une quelconque des revendications précédentes, dans lequel au moins deux encoches de surface (8 ; 26) sont prévues dans ladite région de bordure (48).

11. Clou intramédullaire (10) selon la revendication 10, dans lequel une première encoche de surface (8 ; 26) est positionnée dans une moitié proximale de ladite région de bordure (48), et une seconde encoche de surface (8 ; 26) est positionnée dans une moitié distale de ladite région de bordure (48), et/ou dans lequel une première encoche de surface (8 ; 26) est prévue à une extrémité proximale de ladite région de bordure (48) et/ou une seconde encoche de surface (8 ; 26) est prévue à une extrémité distale de ladite région de bordure (48).

12. Clou intramédullaire (10) selon l'une quelconque des revendications précédentes, dans lequel ladite encoche de surface (26) est prévue sous la forme d'au moins une encoche de surface circonférentielle (26) autour d'une circonférence dudit clou intramédullaire (10), et dans lequel ladite encoche de surface circonférentielle (46) est prévue dans un plan (44) qui est au moins sensiblement perpendiculaire audit axe (28) du clou, ou bien ladite encoche de surface circonférentielle (26) est prévue dans un plan en oblique par rapport à l'axe (28) du clou.

13. Clou intramédullaire (10) selon l'une quelconque des revendications précédentes, dans lequel ladite encoche de surface (8 ; 26) est prévue sur un côté latéral (4) dudit clou intramédullaire (10).

14. Clou intramédullaire (10) selon l'une quelconque des revendications précédentes, dans lequel ladite encoche de surface (8 ; 26) est convenable pour interrompre des lignes de contrainte de traction dans ladite région de bordure (48), induites par une force (F_{L}) agissant sur un point de support pour ladite vis (22) et/ou ladite broche sur ledit premier côté (4) dans une région proximale de ladite première extrémité dudit trou traversant (2).

15. Système à clou intramédullaire (40), en particulier pour l'utilisation à titre de système ostéosynthétique fémoral incluant un clou intramédullaire (10) en accord avec l'une quelconque des revendications précédentes et une vis (22) et/ou une broche, dans lequel ladite vis et/ou ladite broche sont adaptées à être reçues dans ledit trou traversant (2) dudit clou intramédullaire (10) et sont adaptées pour le traitement de fractures osseuses.
